(19) Europäisches Patentamt
European Patent Office
Office européen des brevets

(11) Veröffentlichungsnummer: **0 192 215**
**B1**

# (12) EUROPÄISCHE PATENTSCHRIFT

(45) Veröffentlichungstag der Patentschrift: **27.04.88**

(51) Int. Cl.⁴: **C 07 C 149/267**, C 07 C 49/83, B 41 M 5/18, B 41 M 5/26, G 03 C 1/72

(21) Anmeldenummer: **86101985.9**

(22) Anmeldetag: **17.02.86**

(54) **Neue Tetraphenyldithiolen-Komplexe, unsymmetrisch substituierte Benzoine sowie die neuen Komplexe enthaltende optische Aufzeichnungsmedien.**

(30) Priorität: **20.02.85 DE 3505751**

(43) Veröffentlichungstag der Anmeldung: **27.08.86 Patentblatt 86/35**

(45) Bekanntmachung des Hinweises auf die Patenterteilung: **27.04.88 Patentblatt 88/17**

(84) Benannte Vertragsstaaten: **CH DE FR GB IT LI NL**

(56) Entgegenhaltungen:
**DE-A-2 456 075**
**DE-A-2 908 633**
**DE-A-2 951 341**
**DE-A-3 319 738**
**GB-A-1 263 910**
**US-A-3 816 538**
**US-A-3 875 199**
**US-A-4 320 489**

(73) Patentinhaber: **BASF Aktiengesellschaft, Carl-Bosch- Strasse 38, D-6700 Ludwigshafen (DE)**

(72) Erfinder: **Schrott, Wolfgang, Dr., Bruesseler Ring 45, D-6700 Ludwigshafen (DE)**
Erfinder: **Neumann, Peter, Dr., Franz- Schubert-Strasse 1, D-6908 Wiesloch (DE)**
Erfinder: **Albert, Bernhard, Dr., Riethburgstrasse 13, D-6701 Maxdorf (DE)**
Erfinder: **Thomas, Michael, Dr., Am Wingertsberg 4, D-6719 Weisenheim am Berg (DE)**
Erfinder: **Barzynski, Helmut, Dr., An der Ameisenhalde 49, D-6702 Bad Duerkheim (DE)**
Erfinder: **Schomann, Klaus- Dieter, Dr., Kopernikusstrasse 47, D-6700 Ludwigshafen (DE)**
Erfinder: **Kuppelmaier, Harald, Dr., Kuehler Grund 30a, D-6900 Heidelberg (DE)**

**EP 0 192 215 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Tetraphenyl-dithiolen-Komplexe, unsymmetrisch substituierte Benzoine als Zwischenprodukte zu deren Herstellung sowie ein optisches Aufzeichnungsmedium, das die neuen Tetraphenyl-dithiolen-Komplexe enthält.

Aufzeichnungsmaterialien, die mit Strahlen hoher Energiedichte, z. B. Laserlicht, eine lokal begrenzte Zustandsänderung erfahren, sind bekannt. Mit dieser thermisch ausgelösten Zustandsänderung, z. B. Verdampfen, Änderung des Fließverhaltens oder Ausbleichen, ist eine Änderung der optischen Eigenschaften, z. B. der Absorption durch Änderung des Absorptionsmaximums oder der Extinktion verbunden, welche zur Informations- oder Datenaufzeichnung ausgenutzt werden kann.

Wegen der geringen Größe des Bauelementes, seines geringen Energiebedarfs und der Möglichkeit der direkten Modulation der optischen Ausgangsleistung durch Modulation des elektrischen Antriebstromes eignen sich Festkörper-Injektionslaser, die im nahen Infrarot emittieren, vor allem der AlGaAs-Laser, der im Wellenlängenbereich zwischen etwa 750 und 950 nm arbeitet, besonders gut als Lichtquelle für ein optisches Aufnahmesystem.

Für diese Anwendung ist eine große Anzahl von anorganischen und organischen Materialien bekannt, die in diesem Wellenlängenbereich eine ausreichende Absorption aufweisen, und die ihre optischen Eigenschaften durch Absorption der Strahlung und damit der darin enthaltenen Energie im angegebenen Wellenlängenbereich durch Ablösen, Verdampfen, Schmelzen oder in anderer Weise verändern.

Die bekannten Informationsaufzeichnungsmaterialien bestehen aus einem Träger, auf den dünne Schichten von anorganischen Materialien, z. B. Metalle, Halbmetalle, Legierungen oder Chalkogenglas oder organischen Verbindungen, z. B. IR-Farbstoffe, aufgebracht sind. Die dünnen Schichten werden vor allem durch Aufdampfen im Vakuum oder durch Zerstäubungstechniken erzeugt.

Die Dicke der Schichten sollte so gewählt werden, daß die gesamte eingestrahlte Strahlung absorbiert wird, es sei denn, daß Interferenzphänomene ausgenutzt werden sollen. Der Träger kann aus Glas oder einem geeigneten Kunststoff, z. B. Polycarbonat, Polymethylmethacrylat, Polystyrol, PolystyrolCopolymere, Polyvinylchlorid oder Polymethylpenten, bestehen.

Für die Anwendung als Speichermedium ist es erforderlich, daß sich die amorphen Schichten über längere Zeiträume nicht verändern.

Alterungsprozesse, z. B. Kristallisation oder Ausbleichen durch Licht und Wärme, welche die Morphologie der Speicherschicht verändern, treten bei dünnen, aufgedampften Schichten relativ häufig auf. Neutrale IR-Farbstoffe in Polymerfilen, ionogene IR-Farbstoffe in verlackter Form oder an Polymeren chemisch gebundene IR-Chromophore sollten über längere Zeiträume stabiler sein. Außerdem haben letztere den Vorteil eines wirtschaftlicheren Herstellungsverfahrens.

Optische Aufzeichnungsmedien für den Betrieb mit Halbleiter-Injektionslasern sind in großem Umfang hinsichtlich Trägermaterial, Reflektormaterial und laserlichtempfindlicher Schicht bekannt. Als organische IR-Farbstoffe werden insbesondere Phthalocyaninverbindungen, Methin-Farbstoffe und Quadratsäure-Derivate beschrieben. Azofarbstoff-Komplexe, Anthrachinon- und Triphenylmethanfarbstoffe sowie Pyrylium- und Thiopyryliumsalze sind ebenfalls beschrieben, aber nur eingeschränkt verwendbar, da ihr Absorptionsmaximum in den meisten Fällen für die bekannten Halbleiterlaser zu kurzwellig liegt. Dieses Problem entfällt bei den Dithiolen-Komplexen, die in großer Anzahl bekannt sind [z. B. J.A. McCLEVERTY, Progr. Inorg. Chem. 10, S. 49 - 221 (1968); G.N. SCHRAUZER, Acc. Chem. Res. 2, S. 72 -80 (1969)] und deren Anwendung in optischen Aufzeichnungsmedien beschrieben ist.

In der WO 83/02 428 wird ein optisches Aufzeichnungsmedium beschrieben, das Nickel-Benzodithiolen-Komplexe der Formel

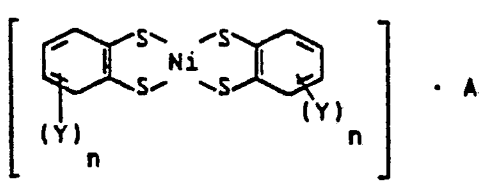 (I)

in der X Wasserstoff, 1-Methyl, 1,4-Dimethyl, 1,2,3,4-Tetramethyl, 1-Chlor und 1,2,3,4-Tetrachlor und A ein Tetraalkylammoniumkation bedeuten, enthält. Diese Komplexe absorbieren zwischen 800 und 950 nm (ε kleiner 17 000).

Aus der DE-A-2 951 341 ist ein, mit einem AlGaAs-Laser beschriebener Aufzeichnungsträger bekannt, der auf einem Glassubstrat eine lichtreflektierende Schicht, z. B. Gold, und eine lichtabsorbierende Schicht trägt. Die Schichten wurden durch Verdampfen im Vakuum auf dem Substrat abgeschieden. Die lichtabsorbierende Schicht besteht aus Dithiolen-Komplexen der Formel

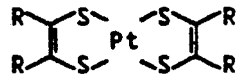 (II),

in der R Phenyl oder substituiertes Phenyl bedeutet.

Ein ähnliches System wird in der US-A-4 320 489 als reversibler optischer Aufzeichnungsträger beschrieben, der auf der Oberfläche als lichtempfindliche Schicht eine Thermoplastschicht aufweist, welche Verbindungen der Formel

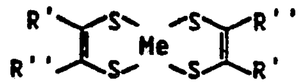 (III)

enthält, in der Me die Metalle Nickel, Palladium oder Platin und R' und R" Alkyl-, Phenyl- oder substituierte Phenylgruppen bedeuten. Angaben über die Qualität des Aufzeichnungsträgers, z. B. in Form von Lese- oder Schreibenergien oder Signal-zu-Rauschen-Verhältnis werden allerdings nicht gemacht.

Bei Anwendung von anderen, aus der Literatur bekannten IR-Farbstoffen, welche der Formel III entsprechen, wurde gefunden, daß diese IR-Farbstoffe in den verwendeten Polymeren nicht ausreichend löslich sind, so daß die damit hergestellten Aufzeichnungsträger zwar beschreibbar waren, jedoch eine unzureichende Qualität aufweisen.

Eigene Versuche zeigten, daß alle bekannten wie auch die vorstehend genannten anderen hochreinen Dithiolenkomplexe sowie Mischungen dieser Verbindungen, die sich durch Vakuumaufdampfungstechniken als dünne Schicht auf einem Träger abscheiden lassen, direkt nach dem Aufdampfen oder nach weiterer thermischer Belastung (Tempern) mehr oder weniger schnell kristallisierten, wodurch diese Farbstoffschichten als Medium eines optischen Aufzeichnungsträgers unbrauchbar sind. Neben den bekannten Nachteilen einer diskontinuierlichen Vakuumverdampfung bei einer Massenproduktion bergen derartige Aufzeichnungsträger ein großes Risiko im Hinblick auf ihre Langzeitstabilität in sich.

In Res. Discl. 21 612/1982 werden Dithiolen-Komplexe der Formel (III) beschrieben, in der $R^1$ unabhängig voneinander für Aryl wie Alkylaryl oder Alkoxyaryl und $R^2$ für Wasserstoff oder gegebenenfalls substituiertes Alkyl und Me für Nickel stehen. Diese Komplexe absorbieren zwischen etwa 700 und 900 nm und weisen gute Löslichkeiten in verschiedenen organischen Solventien auf.

Aufgabe der Erfindung war es, gut lösliche IR-Farbstoffe zu finden, die in den für solche Aufzeichnungsmedien verwendeten Polymeren gut bis leicht löslich sind und sich dementsprechend in hoher Konzentration in dünne Polymerfilme einarbeiten lassen, die weiterhin in den verwendeten Polymeren eine ausreichende Absorption bei der eingestrahlten Lichtwellenlänge aufweisen und die schließlich amorphe Polymerschichten oder Polymerschichten mit kristallinen Bereichen mit einer Größe bilden, die kleiner ist als die Wellenlänge des eingestrahlten Lichts.

Diese Aufgabe wird mit Hilfe der neuen Tetraphenyl-dithiolen-Komplexe gelöst.

Die Erfindung betrifft Tetraphenyl-dithiolen-Komplexe der Formel

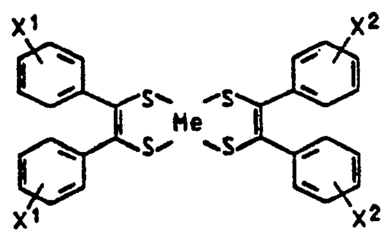 (IV),

in der jeweils ein Rest $X^1$ und $X^2$ für lineares oder verzweigtes $C_5$-$C_{30}$-Alkyl in 4-Stellung, jeweils der andere Rest $X^1$ und $X^2$ für Wasserstoff, $C_1$-$C_{12}$-Alkyl, $C_1$-$C_{12}$-Alkoxy, Fluor, Chlor oder Brom in 2-, 3-oder 4-Stellung, und Me für Nickel, Palladium oder Platin stehen. Die Dithiolen-Komplexe (IV) können in der cis- oder der transForm oder auch als Mischung beider Isomerer vorliegen.

Die Dithiolen-Komplexe gemäß der vorliegenden Erfindung weisen eine sehr hohe molare Absorption (ε bis zu 52 000) im Bereich der Halbleiterlaserlichtwellenlänge von 750 bis 950 nm auf. Sie sind in organischen Lösungsmitteln und in Polymeren gut löslich und ergeben dementsprechend amorphe, nicht kristallisierende Schichten. Die mit Farbstoffen der Formel (IV) erhaltenen Speichermedien weisen eine überlegene Qualität, z. B. einen hohen optischen Kontrast, ein sehr hohes Signal-zu-Rauschen-Verhältnis und eine günstige Schwellenenergie auf.

Als Substituenten $X^1$ und $X^2$ kommen im einzelnen in Betracht:

Jeweils ein $X^1$ und ein $X^2$ steht für $C_5$-$C_{30}$-Alkyl, vorzugsweise für $C_8$-$C_{24}$-Alkyl, wobei diese Alkylgruppen jeweils in 4-Stellung stehen. Die Alkylgruppen können linear oder verzweigt sein. Beispielsweise sind zu

nennen: n-Pentyl, n-Hexyl, Heptyl, n-Octyl, 2-Ethylhexyl, n-Nonyl, n-Decyl, n-Dodecyl, Tridecyl, Tetradecyl, Pentadecyl, Hexadecyl, Octadecyl, Eicosyl, Heneicosyl, Docosyl, Tetracosyl, Pentacosyl, Hexacosyl oder Triacotyl, wobei die Reste von Octyl bis Tetracosyl bevorzugt sind.

Jeweils der andere Rest $X^1$ kann außer Wasserstoff, Chlor, Fluor oder Brom auch $C_1$-$C_{12}$-Alkyl oder $C_1$-$C_{12}$-Alkoxy bedeuten, wobei diese Reste in 2-, 3- oder 4-Stellung stehen können.

Als $C_1$-$C_{12}$-Alkyl, das linear oder verzweigt sein kann, sind z. B. zu nennen: Methyl, Ethyl n- und i-Propyl, n- und i-Butyl, 2-Butyl, tert.-Butyl, Pentyl, n-Hexyl, Heptyl, n-Octyl, 2-Ethylhexyl, Nonyl, n-Decyl n-Dodecyl.

Als $C_1$-$C_{12}$-Alkoxy kommen z. B. in Betracht:

Methoxy, Ethoxy, Propoxy, n-Butoxy, n-Pentoxy, n-Hexoxy, n-Octoxy, 2-Ethylhexoxy, n-Nonoxy, n-Decoxy oder n-Dodecoxy.

Wenn jeweils der andere Rest $X^1$ und $X^2$ für Alkyl oder Alkoxy steht und diese Reste mehr als 2 C-Atome aufweisen, stehen sie vorzugsweise in 4-Stellung.

Vorzugsweise bedeutet jeweils der andere Reste $X^1$ und $X^2$ Methyl, Ethyl, Methoxy, Ethoxy, Fluor, Chlor oder Brom.

Me steht für Nickel, Palladium oder Platin. Aus anwendungstechnischen Gründen sind die Platinkomplexe bevorzugt, da diese überlegene optische Eigenschaften aufweisen und in den für Aufzeichnungsmedien verwendeten Polymeren sehr gut löslich sind und dementsprechend hervorragende Aufzeichnungsmedien ermöglichen.

Die Komplexverbindungen (IV) werden aus Benzoinen nach an sich bekannten Verfahren (G. N. Schrauzer et al., J.A.C.S. 87 (1965), S. 1483 -1489) durch Schwefelung und anschließende Metallsalzfällung im Eintopf-Verfahren hergestellt:

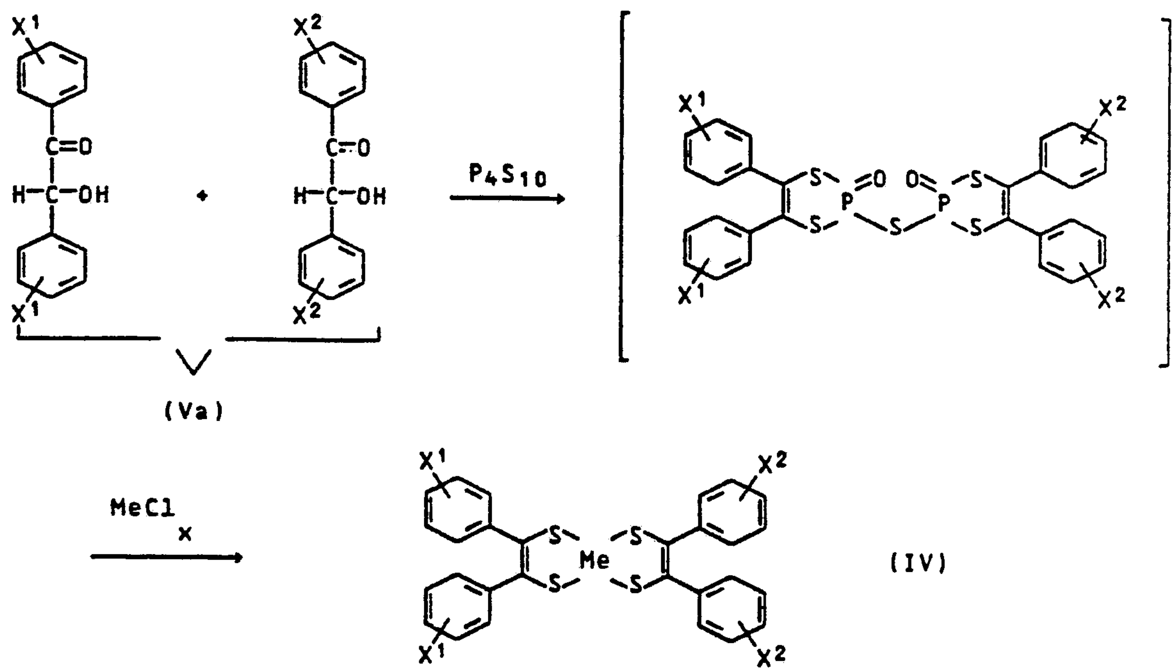

(Va)
(IV)

Sie kann auch aus den entsprechenden Benzilverbindungen, allerdings in deutlich schlechteren Ausbeuten, erhalten werden.

Als Ausgangsmaterialien für die Dithiolen-Komplexe der Formel (IV) dienen die neuen unsymmetrisch substituierten Benzoine der Formel (V)

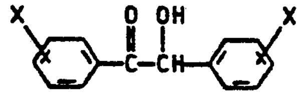
(V),

in der ein Rest X für lineares oder verzweigtes $C_5$-$C_{30}$-Alkyl in 4-Stellung und der andere Rest X für Wasserstoff, $C_1$-$C_{12}$-Alkyl, $C_1$-$C_{12}$-Alkoxy, Fluor, Chlor oder Brom in 2-, 3- oder 4-Stellung steht.

Bevorzugt sind solche Benzoine der Formel (V), in der ein Rest X $C_8$-$C_{24}$-Alkyl und der andere Rest X Methyl, Ethyl, Methoxy, Ethoxy, Fluor, Chlor oder Brom bedeutet.

Die Reste X in Formel (V) sind identisch mit den Resten $X^1$ und $X^2$ in den Formeln (IV) und (Va).

Die erfindungsgemäßen Benzoine der Formel (V), die außerdem wertvolle Zwischenprodukte für die Synthese von Pflanzenschutzmitteln, Pharmazeutika und Farbstoffen darstellen sowie als Additive für Kunststoffe dienen, werden nach an sich bekannten Verfahren hergestellt. Sie werden entweder aus

gegebenenfalls substituiertem Phenylglyoxal und einem substituierten Aromaten [Gleichung (1)] oder aus einem gegebenenfalls substituierten Phenylacetylchlorid und einem substituierten Aromaten mit nachfolgender Oxidation [Gleichung (2)] erhalten:

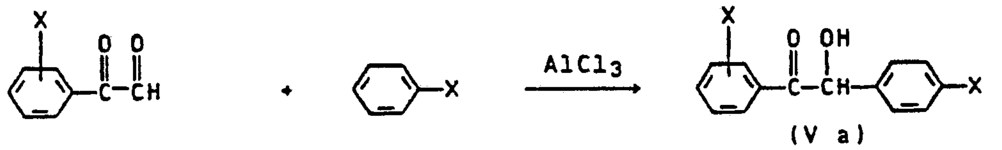


(V a)

(1)

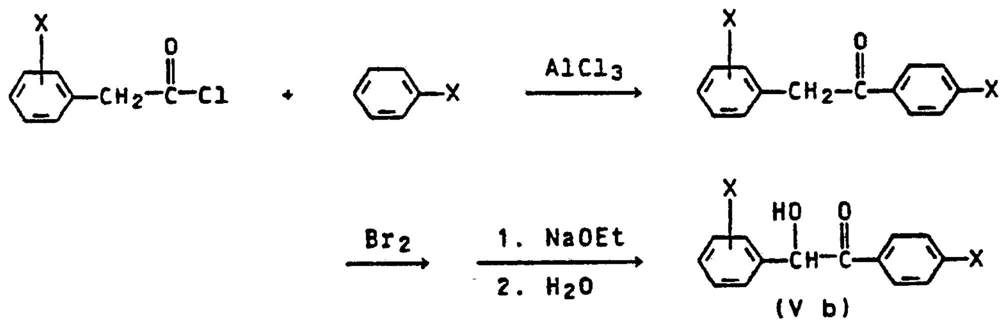


(V b)

(2)

Die neuen Benzoine (V) sind mittels Benzoinkondensation nur in schlechten Ausbeuten oder gar nicht zugänglich.

Die für die Verfahrensvariante (1) als Ausgangsstoffe benötigten ringsubstituierten Phenylglyoxale können aus den entsprechenden Acetophenonen durch Selendioxidoxidation analog der Arbeitsvorschrift in Organic Synthesis, Coll. Vol. 2 (1943), S. 509 hergestellt werden. Die Friedel-Crafts-Acylierungen von substituierten Phenylglyoxalen [Verfahrensvariante (1)] oder ringsubstituierten Phenylessigsäurechloriden [Verfahrensvariante (2)] werden nach modifizierten Arbeitsvorschriften aus Organikum, Berlin, 12. Aufl. 1973, S. 354 durchgeführt (vergl. die Angaben im folgenden unter A.1 und A.2). Die Herstellung der Benzoine (V b) erfolgt durch Bromierung der Desoxybenzoine und anschließende Hydrolyse nach einer modifizierten Arbeitsvorschrift von S. S. Jenkins, J. Am. Chem. Soc. 56 (1934), Seite 682, (vergl. A.2).

Die Erfindung betrifft außerdem optische Aufzeichnungsmedien aus einem Träger und einer gegenüber Laserlicht empfindlichen Farbstoff enthaltenden Schicht aus einem thermoplastischen Polymeren, wobei das thermoplastische Polymere mindestens einen Tetraphenyl-dithiolen-Komplex der Formel

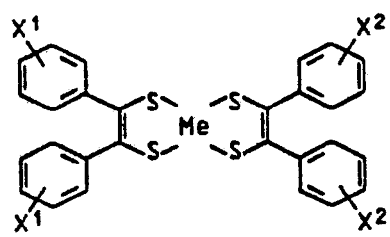

(IV)

enthält, in der jeweils ein Rest $X^1$ und $X^2$ für lineares oder verzweigtes $C_5$-$C_{30}$-Alkyl, in 4-Stellung, jeweils der andere Rest $X^1$ und $X^2$ für Wasserstoff, $C_1$-$C_{12}$-Alkyl, $C_1$-$C_{12}$-Alkoxy, Fluor, Chlor oder Brom in 2-, 3- oder 4-Stellung und Me für Nickel, Palladium oder Platin stehen.

Die bevorzugten Aufzeichnungsschichten enthalten Tetraphenyl-dithiolen-Komplexe der Formel (IV), in der jeweils ein Rest $X^1$ und $X^2$ für $C_8$-$C_{24}$-Alkyl steht und jeweils der andere Rest $X^1$ und $X^2$ die oben angegebene Bedeutung hat.

Insbesondere sind solche Komplexe bevorzugt, in denen jeweils der andere Rest $X^1$ und $X^2$ für Methyl, Ethyl, Methoxy, Ethoxy, Fluor, Chlor oder Brom steht.

Ganz besonders bevorzugte Aufzeichnungsmedien enthalten die Komponenten der Formel (IV) in Form der Platinkomplexe.

Die erfindungsgemäßen Aufzeichnungssysteme haben bei der Wellenlänge der Halbleiterlaser von ca. 750 bis 950 nm eine sehr hohe Absorption. Die Polymerschichten können auf eine lichtreflektierende Schicht so aufgebracht werden, daß sich glatte Absorptionsschichten von optischer Qualität ergeben, in die die zu speichernde Information mit hohem Signal-zu-Rauschen-Verhältnis eingeschrieben werden kann.

Die Aufzeichnungsmedien gemäß der vorliegenden Erfindung sind mit einem Halbleiter-Laser beschreibbar und lesbar. Aufzeichnungsmedien gemäß der vorliegenden Erfindung sind gegenüber atmosphärischen Einflüssen und Tageslicht sehr stabil.

Aufgrund der hohen Lichtabsorption der Farbstoffe sind die erfindungsgemäßen Aufzeichnungsmedien sehr empfindlich gegenüber dem Licht des GaAlAs-Halbleiter-Lasers.

Der Aufbau der Aufzeichnungsmedien ist an sich bekannt.

Zwischen der lichtabsorbierenden Schicht und dem Träger kann eine reflektierende Schicht vorhanden sein, so daß das eingestrahlte und durch die farbige Schicht wandernde Licht (so weit es nicht absorbiert wird) an der Reflektorschicht reflektiert wird und nochmals durch die gefärbte Schicht wandert.

Die Belichtung kann auch durch ein transparentes Substrat erfolgen. Als Schichtfolge kommt dann in Frage: Substrat-Absorberschicht- gegebenenfalls Reflektorschicht.

Die lichtreflektierende Schicht sollte so beschaffen sein, daß diese das zur Aufnahme und zur Abtastung verwendete Licht möglichst quantitativ reflektiert. Geeignete lichtreflektierende Materialien sind z. B. Aluminium, Rhodium, Gold, Zinn, Blei, Wismut, Kupfer und dielektrische Spiegel. Die Dicke der lichtreflektierenden Schicht soll so groß sein, daß diese das zur Aufnahme oder zum Abtasten benutzte Licht möglichst vollständig reflektiert.

Für diesen Zweck sind Spiegel mit geringer Wärmeleitfähigkeit vorteilhaft. Träger, bzw. die Licht reflektierende Schicht müssen eine optisch glatte, ebene Oberfläche aufweisen und an der Oberfläche so beschaffen sein, daß die absorbierende Schicht darauf fest haftet. Um die Oberflächenqualität und Adhäsionsphänomene günstig zu beeinflussen kann der Träger und/oder der Reflektor mit einer Glättungsschicht aus einem duroplastischen oder thermoplastischen Material versehen werden.

Das Aufbringen von metallischen Reflektionsschichten erfolgt vorzugsweise unbekannter Weise durch Aufdampfen im Vakuum oder auch durch Aufbringen geeigneter Metallfolien auf den Träger. Das Aufbringen der erfindungsgemäßen, gegen Laserlicht empfindlichen Schicht erfolgt vorzugsweise durch Aufschleudern von gelöstem oder dispergiertem Farbstoff in Gegenwart von Bindemitteln. Auch Aufrakeln oder Tauchen kommen als Verfahren zur Herstellung der Schichten in Betracht.

Für das Aufbringen der Absorptionsschichten aus Lösung bereitet man in einem geeigneten Lösungsmittel, wie Methylenchlorid, Chloroform, Tetrachlorkohlenstoff, Aceton, Methylethylketon, Cyclohexanon, Toluol, Acetonitril, Essigester, Methanol oder Mischungen dieser Lösungsmittel, eine Lösung oder gegebenenfalls eine Dispersion des Farbstoffs oder Farbstoffgemisches und des Polymeren und setzt gegebenenfalls ein Bindemittel zu.

Als Bindemittel kommen entweder durch Strahlung oder Wärme härtbare Harze, z. B. Photopolymere, Silikonharze sowie Epoxidharze oder thermoplastische Kunststoffe infrage.

Bevorzugt sind thermoplastische Kunststoffe mit keinem oder nur sehr geringem kristallinen Anteil und Glastemperaturen von > 35°C, insbesondere > 75°C. Darüberhinaus müssen die Bindemittel wie Harze oder thermoplastische Kunststoffe mit den erfindungsgemäßen Dithiolenverbindungen (IV) gut verträglich sein. Geeignet sind beispielsweise wasserunlösliche Bindemittel mit hohem Lösungsvermögen für die Dithiolenverbindungen, wie (Meth)-acrylatpolymere und -copolymere, Polystyrolhomo-und -copolymerisate, Polyvinylcarbazol, Polyvinylestercopolymere, Polyvinylchlorid und Celluloseester.

Die Farbstoffzubereitung wird dann durch Rakeln oder Tauchen, vorzugsweise aber durch Aufschleudern auf ein vorher gereinigtes oder vorbehandeltes ("subbing-layer") Substrat aufgebracht und die Schicht an der Luft getrocknet oder gehärtet. Der Film kann auch im Vakuum, bei erhöhter Temperatur oder gegebenenfalls mit Strahlung, getrocknet bzw. gehärtet werden.

Je nach Systemaufbau wird zuerst die Farbstoff-in-Polymer-Schicht und dann der Reflektor aufgebracht oder umgekehrt verfahren. Auf das Aufbringen von Zwischen- und Schutzschichten oder einer reflektierenden Schicht kann gegebenenfalls verzichtet werden.

Sofern die Farbstoff-in-Polymer-Schicht keine ausreichende mechanische Stabilität aufweist, kann diese mit einer transparenten Schutzschicht überzogen werden. Hierzu bieten sich eine Reihe von Polymeren an, welche durch Aufschleudern, Aufrakeln oder Tauchen von gelösten Polymeren oder durch Aufdampfen im Vakuum, insbesondere von fluorierten Polymeren, eine Schutzschicht bilden können.

Wenn das System (Datenspeicher) aus zwei gleichen oder verschiedenen Aufzeichnungsmedien in Form eines "sandwich" aufgebaut wird, kann auf eine Schutzschicht verzichtet werden. Neben größerer mechanischer und rotationsdynamischer Stabilität bietet der "Sandwich"-Aufbau den Vorteil der doppelten Speicherkapazität.

Auf die Schutz- und/oder Zwischenschichten kann bei ausreichender Qualität des optischen Aufzeichnungsmediums verzichtet werden. Wenn auf Zwischenschichten nicht verzichtet werden kann, muß unter Berücksichtigung des Brechungsindex des dazu verwendeten Materials und der verwendeten Laserlichtwellenlänge deren Schichtdicke so gewählt werden, daß keine störenden Interferenzen auftreten können.

Die beim Absorbieren des Laserlichtes entstehende Wärme führt zu einem radial nach außen gerichteten Fließen des Thermoplastes und somit zur Ausbildung von kantenscharfen "Löchern" wodurch ein ausgezeichnetes Signal/Rausch-Verhalten erzielt wird.

Die Erfindung soll durch die folgenden Beispiele zusätzlich erläutert werden. Die Prozentangaben beziehen sich auf das Gewicht.

Die Tetraphenyl-dithiolen-Komplexe werden im folgenden auch als Bis-thiobenzil-Komplexe bezeichnet.

## A. Herstellung der Benzoinverbindungen (V)

### A.1 Darstellung von 4'-n-Octylbenzoin (Verbindung V.1)

[Verfahren (1)]

57,0 g (0,30 mol) n-Octylbenzol werden in 800 ml Schwefelkohlenstoff gelöst, auf 0°C abgekühlt, 66,5 g (0,50 mol) Aluminiumchlorid zugefügt und unter kräftigem Rühren bei 5 bis 10°C eine Lösung aus 40,0 g (0,30 mol) Phenylglyoxal und 50 ml Schwefelkohlenstoff zugetropft. Die gelb bis orange gefärbte Reaktionsmischung wird ohne Kühlen 15 Stunden nachgerührt, dann vorsichtig auf 1 l halbkonz. eiskalte Salzsäure gegossen und gut verrührt. Die Phasen werden getrennt, die organische Phase mit 300 ml Wasser ausgeschüttelt, die wäßrigen Phasen mit 300 ml Methylenchlorid extrahiert und die vereinigten organischen Phasen über Natriumsulfat getrocknet. Nach Filtrieren und Abdestillieren des Lösungsmittels bleiben 75,0 g (≙ 77 % d. Th.) Rohprodukt zurück, das ein Schmelzintervall von 85 - 89°C aufweist (Gehalt nach GC: 95 %). Umkristallisieren aus Ethanol liefert analysenreines 4'-n-Octylbenzoin.

Schmp.: 90 - 91°C (EtOH)

| | | | | |
|---|---|---|---|---|
| $C_{22}H_{28}O_2$ (324,5) | ber: | C 81,5 | H 8,6 | O 9,9 % |
| | gef: | C 81,5 | H 8,6 | O 10,1 % |

IR: ν = 1673 (C=O), 2842 S, 2918 (C-H), 3370, 3415 (O-H) $cm^1$.
MS (70eV): m/e = 324 (M⊕), 219 (100 %, M⊕-$C_6H_5$-C=O), 105 ($C_6H_5$-CO⊕).

### A.2 Darstellung von 4-n-Octylbenzoin (Verbindung V.2)

[Verfahren (2)]

57,0 g (0,30 mol) n-Octylbenzol werden in 500 ml Schwefelkohlenstoff gelöst, bei 0°C werden 45,0 g (0,34 mol) Aluminiumchlorid zugegeben und unter Rühren bei 0 bis 5°C eine Lösung aus 46,4 g (0,30 mol) Phenylessigsäurechlorid und 50 ml Schwefelkohlenstoff zugetropft. Die Reaktionsmischung wird noch 2 Stunden bei 0 bis 5°C, dann 15 Stunden ohne Kühlen nachgerührt. Die Reaktionsmischung wird vorsichtig auf 1 l halbkonz. eiskalte Salzsäure gegossen. Die Phasen werden getrennt, die organische Phase mit 300 ml Wasser ausgeschüttelt, die wäßrigen Phasen dreimal mit je 300 ml Methylenchlorid extrahiert und die vereinigten organischen Phasen über Natriumsulfat getrocknet. Nach dem Filtrieren und Abdestillieren des Lösungsmittels bleiben 92 g (= 100 % d. Th.) Desoxybenzoin als farblose Kristallmasse zurück, die nach DC (Kieselgel/$CH_2Cl_2$: R ≈ 0,95: Toluol: R ≈ 0,61) für die anschließende Bromierung eine ausreichende Reinheit aufweist.

Bei der Wiederholung dieser Arbeitsvorschrift in anderen Ansatzgrößen werden Rohausbeuten zwischen 70 und 90 Prozent erhalten. In diesen Fällen ist einmaliges Umkristallisieren aus Ethanol vor der anschließenden Bromierung notwendig.

120 g (0,35 mol) 4-n-Octyldesoxybenzoin werden in 500 ml 1,2,2-Trichlor-1,1,2-trifluorethan gelöst. Unter Bestrahlung mit UV-Licht werden 16 ml (56 g, 0,35 mol) Brom innerhalb einer Stunde so zugetropft, so daß sich die Lösung ständig entfärbt. Anschließend wird die Reaktionslösung eine Stunde zum Sieden erhitzt und der vollständige Umsatz durch DC geprüft. Das Lösungsmittel wird vollständig abgezogen: Rückstand: 140 g Rohprodukt. Dieses wird in 100 ml Ethanol gelöst und bei Raumtemperatur unter Rühren 56,7 g (1,05 mol) Natriummethanolat in 200 ml Ethanol gelöst zugetropft, wobei die Innentemperatur bis auf 35°C ansteigt. Das Reaktionsgemisch wird 15 h bei Raumtemperatur nachgerührt, dann auf 1 l halbkonzentrierte Salzsäure ausgetragen und etwa 30 Minuten nachgerührt. Die Fällung wird abfiltriert, mit Wasser gewaschen und aus Petrolether (60 - 80°C) umkristallisiert. Ausbeute: 103,2 g (91 %) 4-n-Octylbenzoin (Schmp.: 65 bis 70°C), die nach DC nur geringfügig verunreinigt sind. Durch Säulenchromatographie an Kieselgel 60 mit Methylenchlorid als Laufmittel wurden 70 g (62 %) analysenreines 4-n-Octylbenzoin erhalten (DC: Kieselgel/$CH_2Cl_2$: R = 0,78; Toluol: R = 0,18).

Schmp.: 74 - 75°C

| | | | | |
|---|---|---|---|---|
| $C_{22}H_{28}O_2$ (324,5) | ber.: | C 81,5 | H 8,6 | O 9,9 % |
| | gef.: | C 81,2 | H 8,6 | O 10,0 % |

IR: ν = 1676, 1683 (C=O), 2851, 2923 (C-N), 3350, 3429 (O-H) $cm^{-1}$.
MS (70eV): m/e = 324 (M⊕), 105 (100 %, M⊕-$C_8H_{17}$ 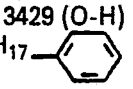 ).

Nach den unter A.1 und A.2 beschriebenen Verfahren (1) und (2) wurden die in der Tabelle 1 a zusammengestellten Benzoine V hergestellt (Ansatzgrößen: 0,05 bis 1,5 Mol) und in der Tabelle 1 b charakterisiert.

**Tabelle 1 a:**

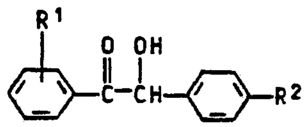 (V)

| Verbindung | $R^1$ | $R^2$ | Ausbeute[1)] (% d. Th) | Herstellungsverfahren |
|---|---|---|---|---|
| V. 1 | H | 4-n-$C_8H_{17}$ | 77 | A |
| V. 2 | 4-n-$C_8H_{17}$ | H | 91 | B |
| V. 3 | H | 4-n-$C_5H_{11}$ | 93 | A |
| V. 4 | 4-n-$C_5H_{11}$ | H | 90 | B |
| V. 5 | 4-n-$C_6H_{13}$ | H | 95 | B |
| V. 6 | 4-n-$C_7H_{15}$ | H | 55 | B |
| V. 7 | 4-n-$C_9H_{21}$ | H | 90 | B |
| V. 8 | 4-n-$C_{10}H_{23}$ | H | 95 | B |
| V. 9 | 4-n-$C_{12}H_{25}$ | H | 96 | B |
| V. 10 | 4-n-$C_{14}H_{29}$ | H | 81 | B |
| V. 11 | 4-n-$C_{15}H_{31}$ | H | 75 | B |
| V. 12 | 2-$CH_3$ | 4-n-$C_8H_{17}$ | 59 | A |
| V. 13 | 3-$CH_3$ | 4-n-$C_8H_{17}$ | 65 | A |
| V. 14 | 4-$CH_3$ | 4-n-$C_8H_{17}$ | 90 | A |
| V. 15 | 4-$CH_3$ | 4-n-$C_{12}H_{25}$ | 83 | A |
| V. 16 | 3-$C_2H_5$ | 4-n-$C_8H_{17}$ | 69 | A |
| V. 17 | 4-$C_2H_5$ | 4-n-$C_8H_{17}$ | 85 | A |
| V. 18 | 2-F | 4-n-$C_8H_{17}$ | 72 | A |
| V. 19 | 3-F | 4-n-$C_8H_{17}$ | 84 | A |
| V. 20 | 4-F | 4-n-$C_6H_{13}$ | 75 | A |
| V. 21 | 4-F | 4-n-$C_7H_{15}$ | 79 | A |
| V. 22 | 4-F | 4-n-$C_8H_{17}$ | 95 | A |
| V. 23 | 4-F | 4-n-$C_9H_{19}$ | 70 | A |
| V. 24 | 4-F | 4-n-$C_{10}H_{21}$ | 82 | A |
| V. 25 | 4-F | 4-n-$C_{12}H_{25}$ | 70 | A |
| V. 26 | 4-F | 4-n-$C_{14}H_{29}$ | 75 | A |
| V. 27 | 4-F | 4-n-$C_{15}H_{31}$ | 77 | A |
| V. 28 | 2-Cl | 4-n-$C_8H_{17}$ | 70 | A |
| V. 29 | 3-Cl | 4-n-$C_8H_{17}$ | 72 | A |
| V. 30 | 4-Cl | 4-n-$C_8H_{17}$ | 82 | A |
| V. 31 | 4-Cl | 4-n-$C_{12}H_{25}$ | 78 | A |
| V. 32 | 2,4-Cl | 4-n-$C_8H_{17}$ | 62 | A |
| V. 33 | 2-Br | 4-n-$C_8H_{17}$ | 65 | A |
| V. 34 | 3-Br | 4-n-$C_8H_{17}$ | 71 | A |
| V. 35 | 4-Br | 4-n-$C_8H_{17}$ | 87 | A |
| V. 36 | 4-Br | 4-n-$C_{12}H_{25}$ | 80 | A |
| V. 37 | 4-Br | 4-n-$C_{15}H_{31}$ | 83 | A |
| V. 38 | 2-$OCH_3$ | 4-n-$C_8H_{17}$ | 52 | A |
| V. 39 | 3-$OCH_3$ | 4-n-$C_8H_{17}$ | 59 | A |
| V. 40 | 4-$OCH_3$ | 4-n-$C_8H_{17}$ | 80 | A |
| V. 41 | 4-$OCH_3$ | 4-n-$C_{12}H_{25}$ | 88 | A |
| V. 42 | 4-$OC_8H_{17}$ | 4-n-$C_8H_{17}$ | 55 | A |
| V. 43 | 4-$SCH_3$ | 4-n-$C_8H_{17}$ | 45 | A |
| V. 44 | 4-$NO_2$ | 4-n-$C_8H_{17}$ | 37 | A |

1) Die Ausbeuten wurden unter den in A.1 und A.2 angegebenen, jedoch nicht optimierten Bedingungen erzielt.

**Tabelle 1 b:** Charakteristische Daten der neuen Benzoine (V) 1)

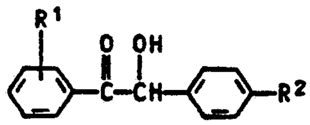

(V)

| Verbindung | Schmp. (°C) | stärkste IR-Banden ν (cm 1) | |
|---|---|---|---|
| V. 1 | 90 | 1678 | MS: m/e = 219 (100 %) M⊕-($C_6H_5$-CO) |
| V. 2 | 74 - 75 | | |
| V. 3 | 94 - 95 | 1675 | |
| V. 4 | 66 - 67 | 1674 | |
| V. 5 | 65 - 66 | 1674 | MS: m/e = 189 (100 %) M⊕-(O=C– 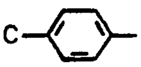 –$C_6H_{13}$) |
| V. 6 | 59 - 60 | 1677 | |
| V. 7 | 53 - 54 | 1677 | |
| V. 8 | 70 - 71 | 1677 | |
| V. 9 | 74 - 75 | 1676 | |
| V. 10 | 91 - 92 | | |
| V. 11 | 88 - 90 | | |
| V. 12 | Öl | | |
| V. 13 | Öl | | |
| V. 14 | 53 - 54 | | |
| V. 15 | 60 - 61 | | |
| V. 16 | Öl | | |
| V. 17 | 64 - 65 | | |
| V. 18 | Öl | | |
| V. 19 | Öl | | |
| V. 20 | 66 - 67 | | 2) |
| V. 21 | 60 - 61 | | 2) |
| V. 22 | 66 - 67 | 1676 | 2) |
| V. 23 | 63 - 64 | | 2) |
| V. 24 | 69 - 70 | | 2) |
| V. 25 | 72 - 73 | | 2) |
| V. 26 | 74 - 75 | | 2) |
| V. 27 | 74 - 75 | | 2) |
| V. 28 | Öl | | |
| V. 29 | Öl | | |
| V. 30 | 84 - 85 | | |
| V. 31 | 79 - 80 | | |
| V. 32 | Öl | | |
| V. 33 | Öl | | |
| V. 34 | Öl | | |
| V. 35 | 92 - 93 | | |
| V. 36 | 80 - 81 | | |
| V. 37 | 64 - 65 | | |
| V. 38 | Öl | | |
| V. 39 | Öl | | |
| V. 40 | 68 - 69 | 1670, 1258, 1171 | $^1$H-NMR: δ = 3,78 ppm ($OCH_3$) |
| V. 41 | 57 - 58 | | |
| V. 42 | Öl | | |
| V. 43 | 86 - 87 | | |
| V. 44 | Öl | | |

1) Durch Säulenchromatographie (LM: $CH_2Cl_2$) gereinigte Benzoine IV lieferten korrekte Elementaranalysen. Die MS-, IR-, 1H-NMR- und 13C-NMR-Spektren stimmen mit den erwarteten Strukturen überein.
2) $^{19}$F-NMR: S = 9,9 ppm (LM: $CDCl_3$, Ref: Fluorbenzol)

**B. Herstellung der Farbstoffe (IV)**

Im folgenden wird je ein Beispiel für die Darstellung eines Nickel-, Palladium- und Platin-Tetraphenyldithiolen-Komplexes nach der Arbeitsvorschrift von G. N. Schrauzer et al., J.A.C.S. 87 (1965), S. 1483 - 1489, die modifiziert wurde, gegeben.

Die Ansatzgrößen wurden zwischen 0,005 und 0,3 mol variiert. Die Rohausbeuten lagen je nach Ansatzgröße zwischen 70 und 100 Prozent. Durch Säulenchromatographie an Kieselgel 60 mit Methylenchlorid als Laufmittel ($R_f \approx 0,9$) wurden hochreine Farbstoffe mit korrekten Elementaranalysen erhalten. Die UV/VIS-, IR-, $^1$H-NMR-, $^{13}$C-NMR- und MS-Spektren (Fragmentierung und Isotopenmuster) stimmen in allen Fällen mit den erwarteten Strukturen überein.

**Beispiel 1**

Bis-(4-n-octyl-dithiobenzil)-nickel (Farbstoff 4 a)

97,2 g (0,30 mol) 4-n-Octylbenzoin (V.1), 400 g (0,30 mol) Ammoniumsulfat und 100 g (0,45 mol) Phosphorpentasulfid werden in 1 l Dioxan aufgeschlämmt und 3 Stunden refluxiert. Nach Abkühlen auf Raumtemperatur wird die Suspension filtriert und mit ca. 100 ml Dioxan gewaschen. Dem Filtrat wird eine Lösung von 72,0 g (0,30 mol) Nickelchloridhexahydrat in 300 ml Wasser zugefügt und 3 Stunden refluxiert. Die Reaktionslösung wird etwa 15 Stunden bei Raumtemperatur nachgerührt, wobei das Robprodukt feinkristallin ausfällt. Das Rohprodukt wird abgesaugt, mit etwas Dioxan, dann mit Wasser und ggf. mit etwas Ethanol gewaschen. Es werden 130 g (56,5 % d. Th.) Produkt erhalten, welches im DC (Kieselgel/$CH_2Cl_2$: $R_f = 0,97$; Toluol: $R_f = 0,72$) einen kleinen Startfleck aufweist. Analysenreines Bis-(4-n-octyl-dithiobenzil)-nickel wird durch Filtrieren einer Methylenchlorid-Lösung über eine kurze Kieselgel-Säule erhalten.
Schmp.: 130 - 131°C

| | | |
|---|---|---|
| $C_{44}H_{52}NiS_4$ (767) | ber.: | Ni 7,7 % |
| | gef.: | Ni 7,3 % |

UV: $\lambda_{max} = 875$ nm ($\varepsilon = 30\,100$) in Toluol

**Beispel 2:**

Bis-(4-n-octyl-dithiobenzil)-palladium (Farbstoff 4 b)

5,20 g (16 mol) 4-n-Octylbenzoin (V.1), 4,4 g (32 mol) Ammoniumsulfat und 7,1 g (32 mol) Phosphorpentasulfid werden in 100 ml Dioxan eingetragen und 2 Stunden refluxiert. Nach Abkühlen auf Raumtenperatur wird die Suspension filtriert, der Rückstand mit wenig Dioxan gewaschen und das Filtrat mit einer Lösung von 2,28 g (7,2 mol) Kaliumtetrachloropalladat(II) in 40 ml Wasser versetzt und 2 Stunden refluxiert. Die Reaktionslösung wird langsam unter Rühren auf Raumtemperatur abgekühlt, mit etwas Dioxan versetzt bis das Produkt kristallin ausfällt und dann abgesaugt.

Das Rohprodukt (4,5 g; 77 %) wird an Kieselgel 60 mit Methylenchlorid chromatographiert.
Ausbeute: 3,8 g (65 % d. Th.) Bis-(4-n-octyl-dithiobenzil)-palladium (Farbstoff 4 b)
UV: $\lambda_{max} = 898$ nm in Methylenchlorid

**Beispiel 3**

Bis-(4-n-octyl-dithiobenzil)-platin (Farbstoff 4 c)

2,33 g (7,2 mol) 4-n-Octylbenzoin (V.1), 1,00 g (7,5 mol) Ammoniumsulfat und 2,44 g (11 mol) Phosphorpentasulfid werden in 20 ml Dioxan eingetragen und 2 Stunden refluxiert. Nach Abkühlen auf Raumtemperatur wird die Suspension filtriert, der Rückstand mit wenig Dioxan gewaschen, das Filtrat mit einer Lösung von 0,50 g (1,2 mol) Kaliumtetrachloroplatinat-(II) in 6,5 ml Wasser versetzt und 2 Stunden refluxiert. Die violette Reaktionslösung wird bei Raumtemperatur mindestens 2 Stunden nachgerührt. Die ölige Reaktionssuspension wird mit etwas Dioxan versetzt, bis eine violette feinkristalline Substanz ausfällt. Der Feststoff wird abgesaugt, in Methylenchlorid aufgenommen und über Kieselgel 60 filtriert. Nach Abziehen des Lösungsmittels und Vakuumtrocknung bleiben 1,10 g violettes Rohprodukt zurück.

Durch Säulenchromatographie an Kieselgel 60 mit Methylenchlorid als Laufmittel erhält man ein dünnschichtchromatographisch reines Produkt (Kieselgel/Toluol: $R_f$: 0,95, $CH_2Cl_2$: $R_f = 0,98$).

Ausbeute: 0,9 g (83 % d. Th.) Bis-(4-n-octyl-dithiobenzil)-platin
Schmp.: 138 - 139°C

| | | |
|---|---|---|
| $C_{44}H_{52}S_4Pt$ (903) | ber.: | Pt 21,6 % |
| | gef.: | Pt 21,0 % |

UV: $\lambda_{max}$ = 815 nm in Methylenchlorid

**Beispiel 4**

Bis-(4-n-octyl-4'-fluor-dithiobenzil)-nickel (Farbstoff 19 a)

6,84 g (20 mmol) 4-n-Octyl-4'-fluor-benzoin (V.21), 4,0 g (30 mol) Ammoniumsulfat und 6,66 g (30 mmol) Phosphorpentasulfid werden in 30 ml Dioxan aufgeschlämmt und 2 Stunden refluxiert. Nach Abkühlen auf Raumtemperatur wird die Suspension filtriert und mit etwa 10 ml Dioxan gewaschen. Dem Filtrat wird eine Lösung von 2,4 g (10 mol) Nickel-(II)-chloridhexahydrat in 10 ml Wasser zugefügt und 2 Stunden refluxiert. Die Reaktionslösung wird dann etwa 2 h bei Raumtemperatur nachgerührt, wobei das Rohprodukt feinkristallin ausfällt. Dieses wird abgesaugt, mit wenig Dioxan, dann mit Wasser und zuletzt mit wenig Ethanol gewaschen. Das Rohprodukt (5,0 g; 62 %) weist im DC (Kieselgel/$CH_2Cl_2$: $R_f$ = 0,90) einen kleinen Startfleck auf. Durch Säulenchromatographie an Kieselgel 60 und Methylenchlorid als Laufmittel werden 2,3 g (28,6 % d. Th.) des analysenreinen Komplexes (19 a) erhalten.

Schmp.: 154 - 155°C

| | | | | | | |
|---|---|---|---|---|---|---|
| $C_{44}H_{50}F_2NiS_4$ (803) | ber.: C 65,8 | H 6,2 | F 4,7 | S 15,9 | Ni 7,4 % |
| | gef.: C 66,1 | H 6,4 | F 4,7 | S 15,6 | Ni 6,8 % |

UV: $\lambda_{max}$ = 870 nm ($\varepsilon$ = 31554) in Toluol; $\lambda_{max}$ = 873 nm ($CH_2Cl_2$).
IR (KBr): $\nu$ = 2924, 2852, 1596, 1504, 1360 (s), 1233, 1144, 890, 829 $cm^{-1}$.
$^{19}F$-NMR ($CDC1_3$): $\delta$ = - 111,0 ppm (Monofluorpentachlorethan).

**Beispiel 5**

Bis-(4-n-octyl-4'-fluor-dithiobenzil)-palladium (Farbstoff 19 b)

2,74 g (8,0 mol) 4-n-Octyl-4'-fluorbenzoin (V.21), 1,1 g (8,3 mol) Ammoniumsulfat und 2,67 (12 mmol) Phosphorpentasulfid werden in 40 ml Dioxan aufgeschlämmt und 2 Stunden refluxiert. Nach Abkühlen auf Raumtemperatur wird filtriert, mit etwa 5 ml Dioxan gewaschen, das Filtrat mit einer Lösung von 1, 27 g (4,0 mmol) Kaliumtetrachloropalladat-(II) in 10 ml Wasser versetzt und 2 Stunden refluxiert. Nach Abkühlen auf Raumtemperatur wird der feinkristalline Niederschlag filtriert, mit wenig Dioxan und Wasser gewaschen. Das Rohprodukt (3,0 g, 88 %) wird in Methylenchlorid aufgenommen und an Kieselgel 60 chromatographiert.

Ausbeute: 1,1 g (32 % d. Th.) Palladiumkomplex (19 b)
UV: $\lambda_{max}$ = 898 nm in Toluol.

**Beispiel 6**

Bis-(4-n-octyl-4'-fluor-dithiobenzil)-platin (Farbstoff 19 c)

0,89 g (2,6 mmol) 4-n-Octyl-4'-fluor-benzoin (V.21), 0,34 g (2,6 mol) Ammoniumsulfat und 0,82 g (5,2 mol) phosphorpentasulfid werden in 20 ml Dioxan aufgeschlämmt und 2 Stunden refluxiert. Nach Abkühlen auf Raumtemperatur wird filtriert, mit einigen Millilitern Dioxan gewaschen, das Filtrat mit einer Lösung von 0,50 g (1,2 mmol) Kaliumtetrachloroplatinat-(II) in 6,5 ml Wasser versetzt und 2 Stunden refluxiert. Nach Abkühlen auf Raumtemperatur wird mit wenig kaltem Dioxan gewaschen, der Rückstand in Methylenchlorid aufgenommen und über eine kurze Kieselgelsäule (Füllhöhe ca. 5 cm) filtriert. Es wurden 0,45 g (40 %) des reinen Platinkomplexes (Kieselgel/$CH_2Cl_2$: $R_f$ = 0,90) erhalten, an dem folgende charakteristischen Daten bestimmt wurden:

Schmp.: 165 - 166°C

| | | | | | | |
|---|---|---|---|---|---|---|
| $C_{44}H_{50}F_2PtS_4$ (939) | ber.: | C 56,2 | H 5,3 | F 4,1 | S 13,6 | Pt 20,8 % |
| | gef.: | C 56,7 | H 5,8 | F 3,9 | S 13,0 | Pt 20,4 % |

UV: $\lambda_{max}$ = 814 nm ($\varepsilon$ = 47210) in Toluol; $\lambda_{max}$ = 817 ($CH_2Cl_2$).
IR (KBr): $\nu$ = 2924, 2852, 1597, 1505, 1407, 1363 (s), 1233, 1148, 887, 829 $cm^{-1}$.
$^{19}$F-NMR ($COCl_3$): $\delta$ = - 111 ppm (Monofluorpentachlorethan).

Entsprechend den Angaben in den Beispielen 1 bis 6 wurden die in der Tabelle 2 a angegebenen Farbstoı der Formel (IV) hergestellt.

Die Farbstoffe sind in der Tabelle 2 b durch das Absorptionsmaximum und gegebenenfalls durch den molaren Extinktionskoeffizienten und den Schmelzpunkt charakterisiert.

Durch Chromatographie über Kieselgel 60 mit Methylenchlorid ($R_f \approx 0,9$) erhält man analysenreine Komplex-Farbstoffe.

**Tabelle 2 a:** Dithiolen-Komplex-Farbstoffe

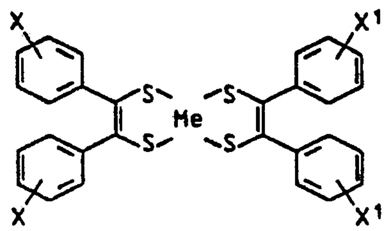

(IV)

| Farbstoff | Me | ein X und ein X¹ | das andere X und das andere X¹ |
|---|---|---|---|
| 1a | Ni | | |
| 1b | Pd | 4-n-$C_5H_{11}$ | H |
| 1c | Pt | | |
| 2a | Ni | | |
| 2b | Pd | 4-n-$C_6H_{13}$ | H |
| 2c | Pt | | |
| 3a | Ni | | |
| 3b | Pd | 4-n-$C_7H_{25}$ | H |
| 3c | Pt | | |
| 4a | Ni | | |
| 4b | Pd | 4-n-$C_8H_{17}$ | H |
| 4c | Pt | | |
| 5a | Ni | | |
| 5b | Pd | 4-n-$C_9H_{19}$ | H |
| 5c | Pt | | |
| 6a | Ni | | |
| 6b | Pd | 4-n-$C_{10}H_{21}$ | H |
| 6c | Pt | | |
| 7a | Ni | | |
| 7b | Pd | 4-n-$C_{12}H_{25}$ | H |
| 7c | Pt | | |
| 8a | Ni | | |
| 8b | Pd | 4-n-$C_{14}H_{29}$ | H |
| 8c | Pt | | |
| 9a | Ni | | |
| 9b | Pd | 4-n-$C_{15}H_{31}$ | H |
| 9c | Pt | | |
| 10a | Ni | | |
| 10b | Pd | 4-n-$C_8H_{17}$ | 2-$CH_3$ |
| 10c | Pt | | |
| 11a | Ni | | |
| 11b | Pd | 4-n-$C_8H_{17}$ | 3-$CH_3$ |
| 11c | Pt | | |
| 12a | Ni | | |
| 12b | Pd | 4-n-$C_8H_{17}$ | 4-$CH_3$ |
| 12c | Pt | | |

| Farbstoff | Me | ein X und ein $X^1$ | das andere X und das andere $X^1$ |
|---|---|---|---|
| 13a | Ni | | |
| 13b | Pd | 4-n-$C_{12}H_{25}$ | 4-$CH_3$ |
| 13c | Pt | | |
| 14a | Ni | | |
| 14b | Pd | 4-n-$C_8H_{17}$ | 3-$C_2H_5$ |
| 14c | Pt | | |
| 15a | Ni | | |
| 15b | Pd | 4-n-$C_8H_{17}$ | 4-$C_2H_5$ |
| 15c | Pt | | |
| 16a | Ni | | |
| 16b | Pd | 4-n-$C_8H_{17}$ | 2-F |
| 16c | Pt | | |
| 17a | Ni | | |
| 17b | Pd | 4-n-$C_8H_{17}$ | 3-F |
| 17c | Pt | | |
| 18a | Ni | | |
| 18b | Pd | 4-n-$C_6H_{13}$ | 4-F |
| 18c | Pt | | |
| 19a | Ni | | |
| 19b | Pd | 4-n-$C_7H_{15}$ | 4-F |
| 19c | Pt | | |
| 20a | Ni | | |
| 20b | Pd | 4-n-$C_8H_{17}$ | 4-F |
| 20c | Pt | | |
| 21a | Ni | | |
| 21b | Pd | 4-n-$C_9H_{19}$ | 4-F |
| 21c | Pt | | |
| 22a | Ni | | |
| 22b | Pd | 4-n-$C_{10}H_{21}$ | 4-F |
| 22c | Pt | | |
| 23a | Ni | | |
| 23b | Pd | 4-n-$C_{12}H_{25}$ | 4-F |
| 23c | Pt | | |
| 24a | Ni | | |
| 24b | Pd | 4-n-$C_{14}H_{29}$ | 4-F |
| 24c | Pt | | |
| 25a | Ni | | |
| 25b | Pd | 4-n-$C_{15}H_{31}$ | 4-F |
| 25c | Pt | | |
| 26a | Ni | | |
| 26b | Pd | 4-n-$C_8H_{17}$ | 2-Cl |
| 26c | Pt | | |
| 27a | Ni | | |
| 27b | Pd | 4-n-$C_8H_{17}$ | 3-Cl |
| 27c | Pt | | |
| 28a | Ni | | |
| 28b | Pd | 4-n-$C_8H_{17}$ | 4-Cl |
| 28c | Pt | | |
| 29a | Ni | | |
| 29b | Pd | 4-n-$C_8H_{17}$ | 2,4-Cl |
| 29c | Pt | | |
| 30a | Ni | | |
| 30b | Pd | 4-n-$C_{12}H_{25}$ | 4-Cl |
| 30c | Pt | | |
| 31a | Ni | | |
| 31b | Pd | 4-n-$C_8H_{17}$ | 2-Br |
| 31c | Pt | | |
| 32a | Ni | | |
| 32b | Pd | 4-n-$C_8H_{17}$ | 3-Br |
| 32c | Pt | | |
| 33a | Ni | | |
| 33b | Pd | 4-n-$C_8H_{17}$ | 4-Br |
| 33c | Pt | | |

| Farbstoff | Me | ein X und ein $X^1$ | das andere X und das andere $X^1$ |
|---|---|---|---|
| 34a | Ni | | |
| 34b | Pd | 4-n-$C_{12}H_{25}$ | 4-Br |
| 34c | Pt | | |
| 35a | Ni | | |
| 35b | Pd | 4-n-$C_{15}H_{31}$ | 4-Br |
| 35c | Pt | | |
| 36a | Ni | | |
| 36b | Pd | 4-n-$C_8H_{17}$ | 2-$OCH_3$ |
| 36c | Pt | | |
| 37a | Ni | | |
| 37b | Pd | 4-n-$C_8H_{17}$ | 3-$OCH_3$ |
| 37c | Pt | | |
| 38a | Ni | | |
| 38b | Pd | 4-n-$C_8H_{17}$ | 4-$OCH_3$ |
| 38c | Pt | | |
| 39a | Ni | | |
| 39b | Pd | 4-n-$C_{12}H_{25}$ | 4-$OCH_3$ |
| 39c | Pt | | |
| 40a | Ni | | |
| 40b | Pd | 4-n-$C_8H_{17}$ | 4-$OC_8H_{17}$ |
| 40c | Pt | | |
| 41a | Ni | | |
| 41b | Pd | 4-n-$C_8H_{17}$ | 4-$SCH_3$ |
| 41c | Pt | | |
| 42a | Ni | | |
| 42b | Pd | 4-n-$C_8H_{17}$ | 4-$NO_2$ |
| 42c | Pt | | |

**Tabelle 2b:** Dithiolen-Komplex-Farbstoffe, physikalische Daten

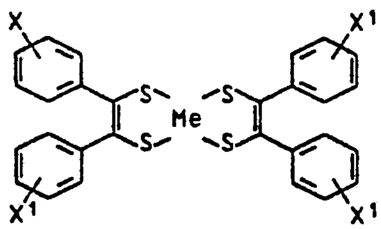

(IV)

$\lambda_{max}$ gemessen in Methylchlorid bzw. Tuloul [1)]

| Farbstoff | $\lambda_{max}$/nm ($\varepsilon \cdot 10^3$) | | Schmp. [0°] |
|---|---|---|---|
| 1a | 871 | (27,4) | 164 - 166 |
| 1b | 897 | | 181 - 183 |
| 1c | 814 | | 185 - 188 |
| 2a | 875 | (30,4) | 148- 150 |
| 2b | 896 | | |
| 2c | 817 | (52,6) | 158 - 159 |
| 3a | 870 | (22,3) | 142 - 143 |
| 3b | 897 | | |
| 3c | 810 | (48,8) | 156 - 157 |
| 4a | 875 | (31,7) | 130 - 131 |
| 4b | 898 | | |
| 4c | 817 | (57,7) | 136 - 139 |
| 5a | 865 | (25,1) | 134 - 135 |
| 5b | 897 | | |
| 5c | 818 | (51,0) | 142 - 143 |
| 6a | 870[1)] | (32,1) | 126 - 127 |
| 6b | 895 | | |
| 6c | 812 | (47,7) | 128 - 132 |
| 7a | 870 | (31,6) | 118 - 120 |
| 7b | 896 | | |
| 7c | 812 | (51,6) | 123 - 127 |

| Farbstoff | $\lambda_{max}$/nm | ($\varepsilon \cdot 10^3$) | Schmp. [0°] |
|---|---|---|---|
| 8a | 868 | (30,0) | 112 - 113 |
| 8b | 895 | | |
| 8c | 811 | (43,8) | 113 - 114 |
| 9a | 870 | (31,3) | 110 - 111 |
| 9b | 896 | | |
| 9c | 811 | (49,9) | 124 - 125 |
| 10a | | | |
| 10b | | | |
| 10c | | | |
| 11a | | | |
| 11b | | | |
| 11c | | | |
| 12a | 883 | (33,3) | 158 - 159 |
| 12b | | | |
| 12c | 822 | (48,9) | 172 - 173 |
| 13a | 885 | (33,5) | 142 - 143 |
| 13b | | | |
| 13c | 828 | (52,5) | 149 - 150 |
| 14a | 880 | (26,1) | Öl |
| 14b | | | |
| 14c | 822 | (48,7) | 108 - 109 |
| 15a | 890 | (33,5) | 179 - 180 |
| 15b | | | |
| 15c | 829 | (50,9) | 194 - 195 |
| 16a | | | |
| 16b | | | |
| 16c | | | |
| 17a | 868 | | |
| 17b | 895 | | |
| 17c | 812 | | |
| 18a | 868 | | |
| 18b | 895 | | |
| 18c | 812 | | |
| 19a | | | |
| 19b | | | |
| 19c | | | |
| 20a | 870[1] | (31,6) | 154 - 155 |
| 20b | 902[1] | | |
| 20c | 815[1] | (47,2) | 160 - 162 |
| 21a | 875 | (29,7) | 138 - 140 |
| 21b | 900 | | |
| 21c | 817 | (47,9) | 144 - 145 |
| 22a | 873 | (31,4) | 140 - 141 |
| 22b | 899 | | |
| 22c | 814 | (48,9) | 152 - 153 |
| 23a | 865 | (26,6) | 128 - 129 |
| 23b | 900 | | |
| 23c | 819 | (42,9) | 140 - 141 |
| 24a | 868 | (29,9) | 125 - 126 |
| 24b | 896 | | |
| 24c | 810 | (47,8) | 134 - 135 |
| 25a | 873 | (28,1) | 121 - 122 |
| 25b | 901 | | |
| 25c | 814 | (47,1) | 125 - 126 |
| 26a | | | |
| 26b | | | |
| 26c | | | |
| 27a | | | |
| 27b | | | |
| 27c | | | |
| 28a | 873 | (30,4) | 161 - 162 |
| 28b | 904 | | |
| 28c | 815 | (51,6) | 192 - 193 |

| Farbstoff | $\lambda_{max}$/nm ($\varepsilon \cdot 10^3$) | | Schmp. [°C] |
|---|---|---|---|
| 29a | | | Öl |
| 29b | | | Öl |
| 29c | | | Öl |
| 30a | 863 | (30,9) | 150 - 151 |
| 30b | 901 | | |
| 30c | 815 | (51,5) | 166 - 168 |
| 31a | | | Öl |
| 31b | | | Öl |
| 31c | 789 | | Öl |
| 32a | | | |
| 32b | | | |
| 32c | | | |
| 33a | 883 | (31,5) | 176 - 177 |
| 33b | 908 | | |
| 33c | 818 | (55,5) | 204 - 205 |
| 34a | 878 | (31,9) | 156 - 157 |
| 34b | 907 | | |
| 34c | 820 | (53,5) | 177 - 178 |
| 35a | 880 | (34,0) | 150 - 151 |
| 35b | 906 | | |
| 35c | 820 | (53,8) | 170 - 171 |
| 36a | | | |
| 36b | | | |
| 36c | | | |
| 37a | | | |
| 37b | | | |
| 37c | | | |
| 38a | 908 | (33,9) | 140 - 145 |
| 38b | | | |
| 38c | 834 | (43,4) | 160 - 165 |
| 39a | 910 | (34,6) | 152 - 154 |
| 39b | | | |
| 39c | 849 | (50,1) | 164 - 165 |
| 40a | | | |
| 40b | | | |
| 40c | | | |
| 41a | | | 157 - 158 |
| 41b | | | |
| 41c | | | 168 - 169 |
| 42a | | | |
| 42b | | | |
| 42c | | | |

**C. Herstellung des optischen Aufzeichnungsmediums**

**Beispiel 7**

Zwei 1,2 mm dicke Polycarbonatscheiben mit einem Durchmesser von 120 mm und einem Innenlochdurchmesser von 15 mm werden mit Alkohol gereinigt und unter Reinraumbedingungen mit einer 0,3 µm starken Glättungsschicht aus einem Photopolymer beschichtet. Nach Aushärtung des Photopolymers mit UV-Licht wurde eine Lösung aus 2 g des Farbstoffs 4 c und 1,3 g eines Copolymers aus Methylmethacrylat/Methacrylsäure (70 : 30) in 200 ml Essigester nach dem Schleuderbeschichtungsverfahren bei 4800 U/min auf die Platten aufgetragen. Nach dem Trocknen betrug die Schichtdicke 0,26 µm. In einer Vakuumbedampfungsapparatur wurde ein Aluminiumspiegel in einer Schichtdicke von 0,03 µm auf die Farbschicht aufgebracht und darauf eine Schutzschicht von 1,2 µm aus Polystyrol in Xylol aufgeschleudert.

Beide Platten werden sandwichartig mit den beschichteten Seiten nach innen über geeignete Abstandsringe zusammengeklebt, so daß ein Luftspalt von 0,4 mm verblieb. Mit einem auf einem Drehtisch montierten AlGaAs-Laser ($\lambda$ = 820 nm) wurden einzelne, etwa 1 φ große Löcher in die aktive Schicht geschrieben. Die Empfindlichkeit der Schicht war besser als 1 nJ/Loch, beim Auslesen der Punkte wurde ein ausgezeichnetes Signal/Rausch-Verhältnis gefunden.

**Patentansprüche**

1. Tetraphenyl-dithiolen-Komplexe der Formel

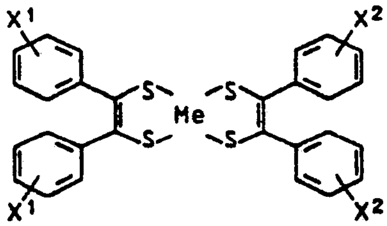

in der jeweils ein Rest $X^1$ und $X^2$ für lineares oder verzweigtes $C_5$-$C_{30}$-Alkyl in 4-Stellung, jeweils der andere Rest $X^1$ und $X^2$ für Wasserstoff, $C_1$-$C_{12}$-Alkyl, $C_1$-$C_{12}$-Alkoxy, Fluor, Chlor oder Brom in 2-, 3-oder 4-Stellung und Me für Nickel, Palladium oder Platin stehen.

2. Tetraphenyl-dithiolen-Komplexe gemäß Anspruch 1, dadurch gekennzeichnet, daß jeweils ein Rest $X^1$ und $X^2$ für lineares oder verzweigtes $C_8$-$C_{24}$-Alkyl steht.

3. Tetraphenyl-dithiolen-Komplexe gemäß Anspruch 1 oder 2, dadurch gekennzeichnet, daß jeweils der andere Rest $X^1$ und $X^2$ für Methyl, Ethyl, Methoxy, Ethoxy, Fluor, Brom oder Chlor steht.

4. Tetraphenyl-dithiolen-Komplexe gemäß Anspruch 1, dadurch gekennzeichnet, daß Me für Platin steht.

5. Unsymmetrisch substituierte Benzoine der Formel

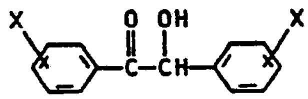 ,

in der ein Rest X für lineares oder verzweigtes $C_5$-$C_{30}$-Alkyl in 4-Stellung und der andere Rest X für Wasserstoff, $C_1$-$C_{12}$-Alkyl, $C_1$-$C_{12}$-Alkoxy, Fluor, Chlor oder Brom in 2-, 3- oder 4-Stellung steht.

6. Unsymmetrisch substituierte Benzoine gemäß Anspruch 5, dadurch gekennzeichnet, daß ein Rest X für lineares $C_8$-$C_{24}$-Alkyl und der andere Rest X für Methyl, Ethyl, Methoxy, Ethoxy, Fluor, Chlor oder Brom steht.

7. Optisches Aufzeichnungsmedium aus einem Träger und einer gegenüber Laserlicht empfindlichen, Farbstoff enthaltenden Schicht aus einem Polymeren, dadurch gekennzeichnet, daß das Polymere mindestens ein Tetraphenyl-dithiolen-Komplex der Formel

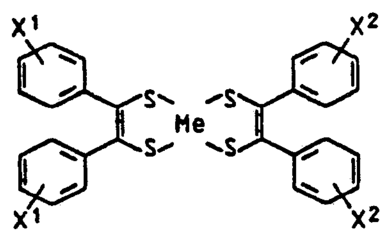

enthält, in der jeweils ein Rest $X^1$ und $X^2$ für lineares oder verzweigtes $C_5$-$C_{30}$-Alkyl in 4-Stellung, jeweils der andere Rest $X^1$ und $X^2$ für Wasserstoff, $C_1$-$C_{12}$-Alkyl, $C_1$-$C_{12}$-Alkoxy, Fluor, Chlor oder Brom in 2-, 3- oder 4-Stellung und Me für Nickel, Palladium oder Platin stehen.

8. Optisches Aufzeichnungsmedium gemäß Anspruch 7, dadurch gekennzeichnet, daß jeweils ein Rest $X^1$ und $X^2$ für lineares oder verzweigtes $C_8$-$C_{24}$-Alkyl steht.

9. Optisches Aufzeichnungsmedium gemäß Auspruch 7, dadurch gekennzeichnet, daß jeweils der andere Rest $X^1$ und $X^2$ für Wasserstoff, Methyl, Ethyl, Methoxy, Ethoxy, Fluor, Chlor oder Brom steht.

10. Optisches Aufzeichnungsmaterial gemäß Anspruch 7, dadurch gekennzeichnet, daß Me für Platin steht.

**Claims**

1. A tetraphenyldithiolene complex of the formula

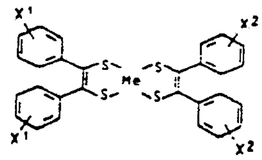

where one radical $X^1$ and one radical $X^2$ are each straight-chain or branched $C_5$-$C_{30}$-alkyl in the 4-position, the other radical $X^1$ and the other radical $X^2$ are each hydrogen, $C_1$-C12 alkyl, $C_1$-$C_{12}$-alkoxy, fluorine, chlorine or bromine in the 2-, 3- or 4-position, and Me is nickel, palladium or platinum.

2. A tetraphenyldithiolene complex as claimed in claim 1, wherein one radical $X^1$ and one radical $X^2$ are each straight-chain or branched $C_8$-$C_{24}$-alkyl.

3. A tetraphenyldithiolene complex as claimed in claim 1 or 2, wherein the other radical $X^1$ and the other radical $X^2$ are each methyl, ethyl, methoxy, ethoxy, fluorine, bromine or chlorine.

4. A tetraphenyldithiolene complex as claimed in claim 1, wherein Me is platinum.

5. An asymmetrically substituted benzoin of the formula

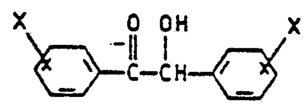

where one radical X is straight-chain or branched $C_5$-$C_{30}$-alkyl in the 4-position, and the other radical X is hydrogen, $C_1$-$C_{12}$-alkyl, $C_1$-$C_{12}$-alkoxy, fluorine, chlorine or bromine in the 2-, 3- or 4-position.

6. An asymmetrically substituted benzoin as claimed in claim 5, wherein one radical X is straight-chain $C_8$-$C_{24}$-alkyl, and the other radical X is methyl, ethyl, methoxy, ethoxy, fluorine, chlorine or bromine.

7. An optical recording medium comprising a base and a dye-containing layer which is sensitive to laser light and consists of a polymer, wherein the polymer contains one or more tetraphenyldithiolene complexes of the formula

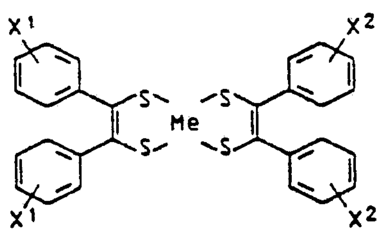

where one radical $X^1$ and one radical $X^2$ are each straight-chain or branched $C_5$-$C_{30}$-alkyl in the 4-position, the other radical $X^1$ and the other radical $X^2$ are each hydrogen, $C_1$-$C_{12}$-alkyl, $C_1$-$C_{12}$-alkoxy, fluorine, chlorine or bromine in the 2-, 3- or 4-position, and Me is nickel, palladium or platinum.

8. An optical recording medium as claimed in claim 7, wherein one radical $X^1$ and one radical $X^2$ are each straight-chain or branched $C_8$-$C_{24}$-alkyl.

9. An optical recording medium as claimed in claim 7, wherein the other radical $X^1$ and the other radical $X^2$ are each hydrogen, methyl, ethyl, methoxy, ethoxy, fluorine, chlorine or bromine.

10. An optical recording medium as claimed in claim 7, wherein Me is platinum.

**Revendications**

1. Complexes de tétraphényldithiolène de formule

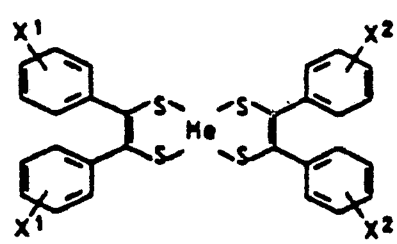

dans laquelle l'un des symboles $X^1$ et $X^2$ est mis pour un radical alkyle en $C_5$-$C_{30}$ à chaîne linéaire ou ramifiée en position 4, les autres symboles $X^1$ et $X^2$ sont mis chacun pour un atome d'hydrogène, un radical alkyle en $C_1$-$C_{12}$, alcoxy en $C_1$-$C_{12}$, un atome de fluor, de chlore ou de brome en position 2, 3 ou 4 et Me est mis pour un atome de nickel, de palladium ou de platine.

2. Complexes de tétraphényldithiolène selon la revendication 1, caractérisés en ce que l'un des symboles $X^1$ et $X^2$ est mis pour un radical alkyle en $C_8$-$C_{24}$ a chaîne linéaire ou ramifiée.

3. Complexes de tétraphényldithiolène selon la revendication 1 ou 2, caractérisé en ce que les autres symboles $X^1$ et $X^2$ sont mis chacun pour un radical méthyle, éthyle, méthoxy, éthoxy, un atome de fluor, de brome ou de chlore.

4. Complexes de tétraphényldithiolène selon la revendication 1, caractérisé en ce que Me est mis pour un atome de platine.

5. Benzoïnes substituées asymétriquement de formule

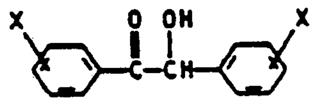

dans laquelle l'un des symboles X est mis pour un radical alkyle en $C_5$-$C_{30}$ à chaîne linéaire ou ramifiée en position 4 et l'autre symbole X est mis pour un atome d'hydrogène, un radical alkyle en $C_1$-$C_{12}$, alcoxy en $C_1$-$C_{12}$, un atome de fluor, de chlore ou de brome en position 2, 3 ou 4.

6. Benzoïnes substituées asymétriquement selon la revendication 5, caractérisées en ce que l'un des symboles X est mis pour un radical alkyle en $C_8$-$C_{24}$ à chaîne linéaire et l'autre symbole X est mis pour un radical méthyle, éthyle, méthoxy, éthoxy, un atome de fluor, de chlore ou de brome.

7. Matériel d'enregistrement optique, se composant d'un support et d'une couche d'un polymère contenant un colorant et sensible à la lumière laser, caractérisé en ce que le polymère contient au moins un complexe de tétraphényldithiolène de formule

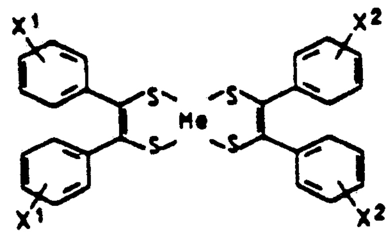

dans laquelle l'un des symboles $X^1$ et $X^2$ est mis pour un radical alkyle en $C_5$-$C_{30}$ à chaîne linéaire ou ramifiée en position 4, les autres symboles $X^1$ et $X^2$ sont mis chacun pour un atome d'hydrogène, un radical alkyle en $C_1$-$C_{12}$, alcoxy en $C_1$-$C_{12}$, un atome de fluor, de chlore ou de brome en position 2, 3 ou 4 et Me est mis pour un atome de nickel, de palladium ou de platine.

8. Matériel d'enregistrement optique selon la revendication 7, caractérisé en ce que l'un des symboles $X^1$ et $X^2$ est mis pour un radical alkyle en $C_8$-$C_{24}$ à chaîne linéaire ou ramifiée.

9. Matériel d'enregistrement optique selon la revendication 7, caractérisé en ce que les autres symboles $X^1$ et $X^2$ sont mis chacun pour un atome d'hydrogène, un radical méthyle, éthyle, méthoxy, éthoxy, un atome de fluor, de brome ou de chlore.

10. Matériel d'enregistrement optique selon la revendication 7, caractérisé en ce que Me est mis pour un atome de platine.